# EUROPEAN PATENT APPLICATION

(11) **EP 3 909 499 A1**
(43) Date of publication of application: **17.11.2021**
(21) Application number: 20175025.4
(22) Date of filing: 15.05.2020
(51) Int. Cl.: A61B 5/00, A61B 5/024, A61B 5/021, A61B 5/08, A61N 1/18, A61N 1/06, A61B 5/145, A61B 5/0476, A61B 5/0488, A61B 5/0205, A61B 5/0402, A61N 5/06

(54) **A WEARABLE MONITORING AND ASSISTANCE SYSTEM**

(71) Applicant: Innovius B.V., 5581 JS Waalre (NL)
(72) Inventor: Serrarens, Alex, 5581 JS Waalre (NL)
(74) Representative: Renkema, Jaap

(57) **Abstract**

The invention relates to a wearable monitoring and assistance system (10) for detecting at least one of physiological and biometrical data of a body of a user and/or for exciting the body locally. The system comprises a housing (20) that is configured to be attached to at least a part (11) of the body and has an ergonomic shape that corresponds to said part of the body. The housing comprises, sensors (24), transducers (27), a processing unit (26), and an antenna (23) that is arranged in a first part (21) of the housing located farthest away from said part, wherein the antenna (23) is configured to avoid being in contact with said part. The system further comprises an energy supply (40) that is arranged outside of the housing, wherein the energy supply is operatively connected with the sensors, the transducers, the processing unit and the antenna.

## Description

### FIELD OF THE INVENTION

The present invention relates to a wearable monitoring and assistance system for detecting at least one of physiological and biometrical data of a body of a user and/or for exciting the body locally with stimuli.

### BACKGROUND OF THE INVENTION

Life expectancy in most countries has been increasing continually over the several few decades thanks to significant improvements in medicine, public health, as well as personal and environmental hygiene. However, increased life expectancy combined with falling birth rates are expected to engender a large aging demographic in the near future that would impose significant burdens on the socio-economic structure of these countries. Therefore, it is essential to develop cost-effective, easy-to-use systems for the sake of elderly healthcare and well-being. Remote health monitoring, based on non-invasive and wearable systems, actuators and modern communication and information technologies offers an efficient and cost-effective solution that allows the elderly to continue to live in their comfortable home environment instead of expensive healthcare facilities. These systems will also allow healthcare personnel to detect important physiological signs of their patients in real time, assess health conditions and provide feedback from distant facilities.

Remote healthcare detection allows people to continue to stay at home rather than in expensive healthcare facilities such as hospitals or nursing homes. It thus provides an efficient and cost-effective alternative to on-site clinical monitoring. Such systems equipped with non-invasive and unobtrusive wearable sensor systems can be viable diagnostic tools to the healthcare personnel for monitoring important physiological signs and activities of the patients in real-time, from a distant facility. Therefore, it is understandable that wearable systems play a critical role.

Wearable monitoring devices can detect and record real-time information about one's physiological condition and motion activities. Wearable sensor system-based health monitoring systems may comprise different types of flexible sensors and transducers that can be integrated into textile fiber, clothes, and elastic bands or directly attached to the human body. The sensors are capable of measuring physiological signs such as electrocardiogram (ECG), electroencephalography (EEG), electromyogram (EMG), heart rate (HR), body temperature, electrodermal activity (EDA), arterial oxygen saturation (SpO2), blood pressure (BP) and respiration rate (RR). In addition, micro-electro-mechanical system (MEMS) based miniature motion sensors such as accelerometers, gyroscopes, and magnetic field sensors are widely used for measuring activity related signals. Continuous monitoring of physiological signals could help to detect and diagnose several cardiovascular, neurological and pulmonary diseases at their early onset. Also, real-time monitoring of an individual's motion activities could be useful in fall detection, gait pattern and posture analysis, or in sleep assessment such as sleep apnea detection in combination with cardiovascular signs for monitoring potential atrial fibrillation. The wearable monitoring systems are usually equipped with a variety of electronic and MEMS sensors, actuators, wireless communication modules and signal processing units. The measurements obtained by the sensors are transmitted to a nearby processing unit using a suitable communication protocol, preferably a low-power and short-range wireless medium.

In order to be used for long-term monitoring purposes, wearable health monitoring systems need to satisfy certain medical and ergonomic requirements. For example, the system needs to be comfortable; the components should be flexible, small in dimensions and must be chemically inert, nontoxic and hypo-allergenic to the human body. In addition, limitation of hardware resources is a major concern for a multi-sensor system where the central node needs to handle a large amount of data coming from different sensor nodes. It also causes significant impact on the system power requirements that needs to be minimized in order to extend the battery life for long-term use. Furthermore, since the device is used for remote monitoring of mobile and free moving persons, the data connectivity of the device with external data and intervention centres must be wireless and must have reliable quality.

There is a need to provide a wearable monitoring and assistance system that detects at least one of physiological and biometrical data of a body of a user and/or for exciting the body locally with stimuli that has an improved ergonomic shape, for example perfectly congruent to wearing locations on the human body. And that it enables improved wireless connectivity and has sufficient energy for long operational intervals between recharging events or its rechargable energy carrier is easily exchangeable/swappable to enable continous use.

### SUMMARY OF THE INVENTION

It is an object of the present invention to provide a wearable monitoring and assistance system for detecting at least one of physiological and biometrical data of a body of a user and/or for exciting the body locally with stimuli that has an improved ergonomic shape, enables improved wireless connectivity as well as sufficient operational energy.

Aspects of the present invention are set out in the accompanying independent and dependent claims. Features from the dependent claims may be combined with features from the independent claim as appropriate and not merely as explicitly set out in the claims.

At least the abovementioned object is achieved by a wearable monitoring and assistance system for detecting at least one of physiological and biometrical data of a body of a user and/or for exciting the body locally with stimuli, the system comprising:
- a housing that is configured to be attached in use of said system to at least a part of the body of the user, the housing having an ergonomic shape that is congruent to said part of the body, wherein the housing comprises:
   µ a number of sensors that are configured to detect said physiological and/or biometrical data of said part of the body,
   • a number of transducers that are configured to excite said part of the body locally with stimuli,
   • a processing unit that is operatively connected to the number of sensors and the number of transducers, wherein the processing unit is configured to generate a signal based on said physiological and/or biometrical data and is configured to generate stimuli for locally exciting said part of the body,
   • an antenna that is adapted to receive the signal from the processing unit,
   • an electronic module that is operatively connected with the number of sensors, the number of transducers, the processing unit and the antenna,
- an energy supply that is operatively connected with the electronic module, wherein the energy supply is arranged outside of the housing,
wherein the housing further comprises a first part that is arranged outwardly protruding from said part of the body and remaining within the perimeter of the said part of the body, and wherein the antenna is integrally attached underneath of the first part thereby maintaining the antenna at a distance from said part of the body and to avoid contact with said part of the body.

The person skilled in the art will appreciate that when the housing that accommodates the antenna is positioned close to at least one of said part of the body and the energy supply, the antenna can be disturbed by at least one of said part of the body and the energy supply. As a result of such disturbance much more power can be required for transmitting electromagnetic waves, e.g. radio waves, via the antenna. This can result in at least one of a reduced lifetime of the energy supply and undesired heat generation, which is undesirable for a wearable system.

The first part may have as much area as possible within the limitations by said part of the body. Therefore, the antenna that is arranged on the first part may take up the largest area within the limitations of said part of the body. In this way, the antenna is always placed as far as possible on the outside of the housing, and claims as much open space as possible within the limitations of said part of the body. Such a configuration of the antenna may prevent interference that is caused by an energy supply while transferring the signal that is generated by the processing unit via the antenna.

Furthermore, the person skilled in the art will appreciate that electromagnetic waves can be attenuated by said part of the body, especially when said part of the body is relatively rich with fluids. An example of a part of the body that is rich with fluids is an earlobe. By arranging the antenna according to the first part of the housing, when the system is in use, to maintain the antenna of the housing at a distance from said part of the body and to avoid contact between the antenna of the housing and said part of the body, disturbance of the antenna by said part of the body can be reduced. Preferably, when the system is in use, the housing is attached to said part of the body to maintain the antenna of the housing at a position as far away as possible from said part of the body and at the outside thereof.

The housing of the system according to the invention has an ergonomic shape. This is to be construed as the housing being configured to be contoured to engage with the at least a part of the body the user in a mating engagement. This mating engagement provides stability regarding the attachment of the housing to said part of the body. As a result of the stable attachment of the housing detecting and/or exciting events by the respective sensors and transducers of the housing can be improved.

The sensors may contain physiological sensors and environmental sensors that detect biometrical data of the body. Transducers for example may contain at least one of a speaker for smart audio outputs and an audio delivery path with the audio interface.

If desired, physiological sensors can be any type of compact sensors for detecting the physiological functioning of the body, such as, but not limited to, sensors for detecting heart rate, pulse rate, breathing rate, blood flow, VO2, VO2max, blood oxygen, blood constituent levels, blood glucose level, heartbeat signatures, cardio-pulmonary health, organ health, metabolism, electrolyte type and concentration, physical activity, caloric intake, caloric metabolism, metabolomics, physical and psychological stress levels and stress level indicators such as delta-, theta-, alpha-, beta-, gamma- brainwaves in combination with heart rate variability and breathing anomalies, physiological and psychological response to therapy, drug dosage and activity (drug dosimetry), physiological drug reactions, drug chemistry in the body, biochemistry, position and balance, body strain, neurological functioning, brain activity, brain waves, blood pressure, cranial pressure, hydration level, auscultatory information, auscultatory signals associated with pregnancy, physiological response to infection, skin and core body temperature, eye muscle movement, blood volume, inhaled and exhaled breath volume, physical exertion, exhaled breath physical and chemical composition, the presence, identity, and concentration of viruses and bacteria, foreign matter in the body, internal toxins, heavy metals in the body, anxiety, fertility, ovulation, sex hormones, psychological mood, sleep patterns, hunger and thirst, hormone type and concentration, cholesterol, lipids, blood panel, bone density, body fat density, muscle density, organ and body weight, reflex response, sexual arousal, mental and physical alertness, sleepiness, auscultatory information, response to external stimuli, swallowing volume, swallowing rate, sickness, voice characteristics, tone, pitch, and volume of the voice, vital signs, head tilt, allergic reactions, inflammation response, auto-immune response, mutagenic response, DNA, proteins, protein or lactate levels in the blood, body hydration, water content of the blood, pheromones, internal body sounds, digestive system functioning, cellular regeneration response, healing response, stem cell regeneration response, and the like. Vital signs can include pulse rate, breathing rate, blood pressure, pulse signature, body temperature, hydration level, skin temperature, and the like.

A physiological sensor may include an impedance plethysmograph for measuring changes in volume within an organ or body (usually resulting from fluctuations in the amount of blood or air it contains). For example, the housing may include an impedance plethysmograph to monitor blood pressure in real-time.

If desired, environmental sensors are adapted to detect the external environment in the vicinity of the body, such as, but not limited to, sensors for monitoring: climate, humidity, temperature, pressure, barometric pressure, pollution, automobile exhaust, soot density, airborne particle density, airborne particle size, airborne particle shape, airborne particle identity, volatile organic chemicals (VOCs), hydrocarbons, polycyclic aromatic hydrocarbons (PAHs), carcinogens, toxins, electromagnetic energy (optical radiation, X-rays, gamma rays, microwave radiation, terahertz radiation, ultraviolet radiation, infrared radiation, radio waves, and the like), EMF energy, atomic energy (alpha particles, beta-particles, gamma rays, and the like), gravity, light properties (such as intensity, frequency, flicker, and phase), ozone, carbon monoxide, greenhouse gases, CO2, nitrous oxide, sulfides, airborne pollution, foreign material in the air, biological particles (viruses, bacteria, and toxins), signatures from chemical weapons, wind, air turbulence, sound and acoustical energy (both human audible and inaudible), ultrasonic energy, noise pollution, human voices, animal sounds, diseases expelled from others, the exhaled breath and breath constituents of others, toxins from others, bacteria and viruses from others, pheromones from others, industrial and transportation sounds, allergens, animal hair, pollen, exhaust from engines, vapors and fumes, fuel, signatures for mineral deposits or oil deposits, snow, rain, thermal energy, hot surfaces, hot gases, solar energy, hail, ice, vibrations, traffic, the number of people in a vicinity of the user, the number of people encountered throughout the day, other earpiece module users in the vicinity of the earpiece module user as to directly communicate with them from distance, coughing and sneezing sounds from people in the vicinity of the user as to avoid virus infections, loudness and pitch from those speaking in the vicinity of the user, and the like.

The processing unit may contain energy cells for signal processing and electronics for the signal forwarding that are received from the sensors and/or the transducers, e.g., via infrared or by radio, e.g. via Bluetooth, WiFi or Zigbee or another wireless, digital or analogue communication protocol. As a result of the place restriction of the processing unit within the housing, the processing unit can be just a telemetry unit as well, i.e. signals sent from it are forwarded to a data exchanging unit.

In an embodiment of the system according to the invention, further comprising a data exchanging unit that is arranged outside of said part of the body and is configured to wirelessly receive the signal from the processing unit via the antenna, and wherein the data exchanging unit is adapted to send an additional signal to the processing unit via the antenna.

In that way, the data exchanging unit may contain electronics for the signal forwarding, e.g., via infrared or by radio, e.g. via Bluetooth, WiFi or Zigbee or another wireless, digital or analogue communication protocol. The data exchanging unit may receive signals from the housing and may process the signal further before they can be used by the user. The data exchanging unit may also transmit an additional signal to the housing, for example, to excite stimuli to said part of the body.

The data exchanging unit may continuously monitor the user's physiological and/or biometrical data signals that are generated from the housing, extract its pattern, predict a trend of the condition and analyze the condition according to a medical expert's knowledge and the user's own health history. In the data exchanging unit, the body temperature may also be monitored, which can be used to help to analyze the user's health condition.

The system may provide the signal and the additional signal that are transmitted using Bluetooth, WiFi (IEEE 802.11 b/g/n/a), Zigbee and/or a further established and normalized digital communication protocol.

In an embodiment of the system according to the invention, the first part of the housing includes a grip structure that accommodates the antenna.

The first part of the housing may be provided with the grip structure. The grip structure is arranged outwardly protruding from said part of the body and remaining within the perimeter of the said part of the body. In that way, the antenna that is positioned inside the grip structure for maximizing reception quality and distance from said part of the body but also facilitates an easy mounting and dismounting of the small housing onto the said part of the body by the user's fingers.

In an embodiment of the system according to the invention, the energy supply is arranged outside of said part of the body.

In this way the system may be provided with the energy supply that is required for performing all functions in the element (sensing, transducing, processing, data storage, data transfer) provide the energy for the housing externally, i.e. from outside the element. The energy supply may transmit the energy via wireless inductive or photovoltaic energy transmission.

In an embodiment of the system according to the invention, the energy supply comprises a battery carrier that is configured to be attached to said part of the body.

In an embodiment of the system according to the invention, the battery carrier is adapted to accommodate at least one battery that is adapted to be swappable.

The energy supply may include batteries that have a metal casing and signal blocking electrolyte inside. The batteries may be relatively large which all together may form a disturbance to the electromagnetic waves, e.g. radio waves, from the antenna. Therefore, more energy could be required to transmit and/or receive signals. By placing the antenna far away from the battery carrier, said disturbance between the antenna and the batteries of the battery carrier can be prevented.

In an embodiment of the system according to the invention, the energy supply is adapted to wirelessly provide energy to the battery carrier.

In this way, the energy supply is provided with a station that is adapted to recharge the at least one battery within the battery carrier.

In an embodiment of the system according to the invention, the battery carrier is connected to the housing via a wired connection system.

In this way, the energy supply is provided with a battery in a housed battery carrier. The battery carrier may be carried by the user. For example, the battery carrier may be attached behind the auricle of the ear or be integrated into a neck-cord attached to the housing. The battery carrier may be connected to housing via the wired connection system. The wired connection system may have at least two wires between the battery carrier and the housing in order to realize energy transmitting. The separation between the battery carrier and the housing may allow the use of relatively larger or odd sized batteries. In that way, the battery carrier may be positioned at a body part with relatively more space or convenience.

In an embodiment of the system according to the invention, the wired connection system is adapted to transmit energy from the battery carrier to the housing in the form of electrical current, magnetic induction or light.

In an embodiment of the system according to the invention, the energy supply is adapted to wirelessly provide energy to the housing.

In an embodiment of the system according to the invention, the number of sensors and the number of transducers are detachable from the housing.

In an embodiment of the system according to the invention, the housing has a second part that in use of the system is arranged to be in contact with said part of the body, wherein the second part has an audio device.

In an embodiment of the system according to the invention, the second part of the housing has at least a part of the number of sensors and of the number of transducers.

In an embodiment of the system according to the invention, the first part of the housing and the second part of the housing are arranged parallel to each other.

In an embodiment of the system according to the invention, wherein the said part the body is configured to define the concha of an ear.

The ear is a relatively immobile platform that does not obstruct the user's movement or vision. The housing may be located inside the opening of the auricle which is defined as concha. The housing that is located at the concha has, for example, access to the inner-ear canal and tympanic membrane (for measuring core body temperature), muscle tissue (for monitoring muscle tension), the pinna and earlobe (for monitoring blood gas levels), the region behind the ear (for measuring skin temperature and galvanic skin response), and the internal carotid artery (for measuring cardiopulmonary functioning). The concha is also at or near the point of exposure to: environmental breathable toxicants of interest (volatile organic compounds, pollution, etc.); noise pollution experienced by the ear; and lighting events for the eye. Furthermore, as the ear canal is naturally designed for transmitting acoustical energy, the concha provides a good location for monitoring internal sounds, such as heartbeat, breathing rate, and mouth motion.

In an exemplary embodiment, the housing may comprise one or more physiological and/or environmental sensors and transducers and may be attached to or near said part of the body via various types of headsets, including wired or wireless headsets. Bluetooth-enabled and/or other personal communication headsets may be configured to incorporate physiological and/or environmental sensors and transducers. Bluetooth headsets are typically lightweight, unobtrusive devices that have become widely accepted socially. Bluetooth headsets may provide a function for the user beyond health monitoring, such as personal communication and multimedia applications, thereby encouraging user compliance with monitoring.

In another exemplary embodiment, said physiological and environmental sensors and transducers may be incorporated into a Bluetooth or other type of headsets include, but are not limited to accelerometers, acoustic sensors, auscultatory sensors, pressure sensors, humidity sensors, colour sensors, light intensity sensors, pressure sensors, etc.

### BRIEF DESCRIPTION OF THE DRAWINGS

Further features and advantages of the invention will become apparent from the description of the invention by way of exemplary and non-limiting embodiments of a system for detecting at least one of physiological and biometrical data of a body of a user and/or for exciting the body locally with stimuli according to the present invention.

The person skilled in the art will appreciate that the described embodiments of the system according to the present invention is exemplary in nature only and not to be construed as limiting the scope of protection in any way. The person skilled in the art will realize that alternatives and equivalent embodiments of the system can be conceived and reduced to practice without departing from the scope of protection of the present invention.

Reference will be made to the figures on the accompanying drawing sheets. The figures are schematic in nature and therefore not necessarily drawn to scale. Furthermore, equal reference numerals denote equal or similar parts. On the attached drawing sheets,
figure 1 illustrates a system with a housing that is attached to a part of a user in accordance with some embodiments; and
figure 2 illustrates inside of the housing in accordance with some embodiments.

### DETAILED DESCRIPTION OF EMBODIMENTS

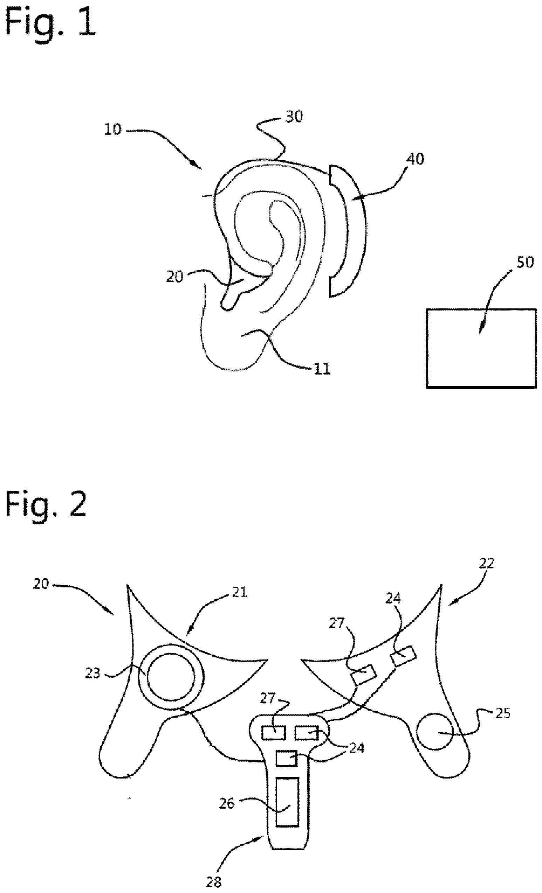

Figure 1 shows system 10 that comprises a housing 20, a data exchanging unit 50, and an energy supply 40 that is connected to the housing 20 via a wired connection system 30. The housing 20 is attached to a part 11 of a user. The data exchanging unit 50 is arranged outside of said part 11 of the body and is configured to wirelessly communicate with the housing 20.

For example, as shown in Figure 1, said part 11 of the body may be an ear. The housing 20 may be attached to the ear which is a relatively immobile platform that does not obstruct the user's movement or vision. The housing 20 located at the ear may have, for example, access to the inner-ear canal and tympanic membrane (for measuring core body temperature), muscle tissue (for monitoring muscle tension), the pinna and earlobe (for monitoring blood gas levels), the region behind the ear (for measuring skin temperature and galvanic skin response), and the internal carotid artery (for measuring cardiopulmonary functioning), etc. The ear is also at or near the point of exposure to: environmental breathable toxicants of interest (volatile organic compounds, pollution, etc.); noise pollution experienced by the ear; and lighting conditions for the eye. Furthermore, as the ear canal is naturally designed for transmitting acoustical energy, the ear may provide a good location for detecting internal sounds, such as heartbeat, breathing rate, and mouth motion.

If desired, the housing 20 may be integrated with an entertainment device for example; headphones, earbuds, audio equipment and gaming devices. Such an entertainment device may offer a platform for performing near-real-time personal health and environmental monitoring in wearable, socially acceptable devices. The capability to unobtrusively detect the user's physiology and/or environment, combined with improved user compliance, may have significant impact on the user's health and environmental exposure.

In that way, the system 10 may give an indication about a link between environmental stressors and personal stress level indicators. For this reason, integrating the housing 20 with the large-scale commercial availability of a low-cost headset devices can enable cost-effective large-scale detections. Such an application of the system 10 may encourage individual-driven health maintenance and may also promote a healthier lifestyle through proper caloric intake and exercise for the user.

In some embodiments, the data exchanging unit 50 may be adapted to receive and/or transmit signals to the housing 20. The data exchanging unit 50 may contain electronics for the signal forwarding, e.g., via infrared or by radio, e.g., via Bluetooth, WiFi or Zigbee or another realization of a wireless, digital or analogous transfer function. The data exchanging unit 50 may receive signals from the housing 20 and may process the signal further before they can be used by the user. The data exchanging unit 50 may also transmit an additional signal to the housing 20, for example, to excite stimuli to said part 11 of the body.

The data exchanging unit 50 may continuously monitor the user's physiological and/or biometrical data signals that are generated from the housing 20, extracts its pattern, predicts the trend of the condition and analyze the condition according to the medical expert knowledge and the user's own health history. In the data exchanging unit 50, the body temperature may also be monitored, which can be used to help to analyze the user's health condition.

If desired the data exchanging unit 50 may be connected to the healthcare center, doctor or family member, for example, through cellular network or any wireless network via PDA or cell phone available in the art. The urgent contact, health information or location can be transmitted as either user request or automatically generated by the system 10. The data exchanging unit 50 may allow the PDA and/or cell phone to display the health information or save the medical data from the system 10 when necessary or required by the user. On the other side, the PDA or cell phone can connect the healthcare center, doctor or family member without distance limitation as long as commercial wireless communication coverage is available.

The energy supply 40 is required for performing all functions in the system 10 (sensing, transducing, processing, data transfer) provide the energy for the housing externally, i.e. from outside the system 10. If desired, the energy supply 40 may contain a battery carrier that accommodates at least one battery that is swappable. As shown in Figure 1, the battery carrier may be attached behind the auricle of the ear 11 of the body. The battery carrier may be connected to the housing 20 via a wired connection system 30. The wired connection system 30 may ensure the energy transmitting between the battery carrier and the housing 20 in the form of electrical current, magnetic induction or light.

If desired the energy supply 40 may contain a station that is adapted to recharge the at least one battery within the battery carrier. Said station may be portable and remote from said part 11 of the body. Said station may transmit the energy to the battery carrier wirelessly.

According to some embodiments, the energy supply 40 may contain a station that wirelessly provides energy directly to the housing 20. In that case, either one of the housing 20 and the station may have a coil for transmitting the energy. The station may include an AC power source that provides an electromagnetic energy via the coils.

Figure 2 shows inside of the housing 20 having a first part 21 and a second part 22. The housing 20 comprises the electronic module 28 that is located between the first part 21 and the second part. The electronic module 28 may operatively connected with antenna 23, sensors 24, transducers 27, and a processing unit 26. The sensors 24 may detect physiological and/or biometrical data of a part (For example, part 11 of Figure 1) of the body. The transducers 27 may excite said part of the body locally with stimuli. The processing unit 26 may be operatively connected to the sensors 24 and the transducers 27 and generate a signal or stimuli based on said physiological and/or biometrical data. The antenna 23 may receive the signal from the processing unit 26.

As shown in Figure 2, the antenna 23 may be located underneath the first part 21, farthest away from said part of the body, thereby avoiding being in contact with said part of the body. In this way, the antenna 23 is always placed as far as possible on the outside, away from said part of the body and claims as much open space as possible within the limitations of the housing 20. Such a configuration of the antenna 23 may prevent interferences that is caused by an energy supply (for example, energy supply 40 of Figure 1), while transferring the signal that is generated by the processing unit via the antenna 23.

The sensors 24 may contain physiological sensors 24 and environmental sensors 24 that detects biometrical data of the body. The transducers 27 may contain a speaker for smart audio outputs, an audio delivery path with the audio interface. At least a part of said sensors 24 and at least a part of said transducers 27 may be arranged on the second part 22 and may seamlessly contact with the skin of a part (for example part 11 of Figure 1) of the body. The second part 22 of housing may also have an audio device 25.

If desired, the physiological sensors 24 can be any compact sensor for detecting the physiological functioning of the body, such as, but not limited to, sensors for detecting: heart rate, pulse rate, breathing rate, blood flow, VO2, VO2max, blood oxygen, blood constituent levels, blood glucose level, heartbeat signatures, cardio-pulmonary health, organ health, metabolism, electrolyte type and concentration, physical activity, caloric intake, caloric metabolism, metabolomics, physical and psychological stress levels and stress level indicators, physiological and psychological response to therapy, drug dosage and activity (drug dosimetry), physiological drug reactions, drug chemistry in the body, biochemistry, position and balance, body strain, neurological functioning, brain activity, brain waves, blood pressure, cranial pressure, hydration level, auscultatory information, auscultatory signals associated with pregnancy, physiological response to infection, skin and core body temperature, eye muscle movement, blood volume, inhaled and exhaled breath volume, physical exertion, exhaled breath physical and chemical composition, the presence, identity, and concentration of viruses and bacteria, foreign matter in the body, internal toxins, heavy metals in the body, anxiety, fertility, ovulation, sex hormones, psychological mood, sleep patterns, hunger and thirst, hormone type and concentration, cholesterol, lipids, blood panel, bone density, body fat density, muscle density, organ and body weight, reflex response, sexual arousal, mental and physical alertness, sleepiness, auscultatory information, response to external stimuli, swallowing volume, swallowing rate, sickness, voice characteristics, tone, pitch, and volume of the voice, vital signs, head tilt, allergic reactions, inflammation response, auto-immune response, mutagenic response, DNA, proteins, protein or lactate levels in the blood, body hydration, water content of the blood, pheromones, internal body sounds, digestive system functioning, cellular regeneration response, healing response, stem cell regeneration response, and the like. Vital signs can include pulse rate, breathing rate, blood pressure, pulse signature, body temperature, hydration level, skin temperature, and the like. A physiological sensor may include an impedance plethysmograph for measuring changes in volume within an organ or body (usually resulting from fluctuations in the amount of blood or air it contains). For example, the housing 20 may include an impedance plethysmograph to monitor blood pressure in real-time.

If desired, the environmental sensors 24 are adapted to detect the external environment in the vicinity of the body, such as, but not limited to, sensors for monitoring: climate, humidity, temperature, pressure, barometric pressure, pollution, automobile exhaust, soot density, airborne particle density, airborne particle size, airborne particle shape, airborne particle identity, volatile organic chemicals (VOCs), hydrocarbons, polycyclic aromatic hydrocarbons (PAHs), carcinogens, toxins, electromagnetic energy (optical radiation, X-rays, gamma rays, microwave radiation, terahertz radiation, ultraviolet radiation, infrared radiation, radio waves, and the like), EMF energy, atomic energy (alpha particles, beta-particles, gamma rays, and the like), gravity, light properties (such as intensity, frequency, flicker, and phase), ozone, carbon monoxide, greenhouse gases, CO2, nitrous oxide, sulfides, airborne pollution, foreign material in the air, biological particles (viruses, bacteria, and toxins), signatures from chemical weapons, wind, air turbulence, sound and acoustical energy (both human audible and inaudible), ultrasonic energy, noise pollution, human voices, animal sounds, diseases expelled from others, the exhaled breath and breath constituents of others, toxins from others, bacteria and viruses from others, pheromones from others, industrial and transportation sounds, allergens, animal hair, pollen, exhaust from engines, vapors and fumes, fuel, signatures for mineral deposits or oil deposits, snow, rain, thermal energy, hot surfaces, hot gases, solar energy, hail, ice, vibrations, traffic, the number of people in a vicinity of the user, the number of people encountered throughout the day, other earpiece module users in the vicinity of the earpiece module user, coughing and sneezing sounds from people in the vicinity of the user, loudness and pitch from those speaking in the vicinity of the user, and the like.

In some embodiments, the processing unit 26 may be arranged in the electronic module 28 and may contain energy cells for signal processing and electronics for the signal forwarding, e.g., via infrared or by radio, e.g., via Bluetooth, WiFi or Zigbee or another wireless, digital or analogue communication protocol. As a result of the place restriction of the processing unit 26 within the housing 20 can be just a telemetry unit as well, i.e. signals sent from it are forwarded to a data exchanging unit (for example, data exchanging unit 50 of Figure 1).

For example, in the case of wireless disconnections, the processing unit 26 may not transmit the signal to the exchanging unit (for example, data exchanging unit 50 of Figure 1). In that case, the electronic unit 28 may contain a data storage element for storing the data and the signals.

The present invention can be summarized as relating to a wearable monitoring and assistance system 10 for detecting at least one of physiological and biometrical data of a body of a user and/or for exciting the body locally. The system comprises a housing 20 that is configured to be attached to at least a part 11 of the body and has an ergonomic shape that corresponds to said part of the body. The housing comprises, sensors 24, transducers 27, a processing unit 26, and an antenna 23 that is arranged in a first part 21 of the housing located farthest away from said part, wherein the antenna 23 is configured to avoid being in contact with said part. The system further comprises an energy supply 40 that is arranged outside of the housing, wherein the energy supply is operatively connected with the sensors, the transducers, the processing unit and the antenna..

It will be clear to a person skilled application the system is not limited to the healthcare monitoring systems but also personal sports training systems, healthy ageing systems, industrial communication and worker safety monitoring systems, office communication and vitality monitoring systems of deskbound professionals, educational systems, smart home,lifestyle and mindfulness systems, and the like. Additionally, the system may be able to be connected to voice assistant systems such as Alexa, Siri, Cortana, Google Assistant as to further extend its functionality using external services.

It will be also clear to a person skilled in the art that the scope of the present invention is not limited to the examples discussed in the foregoing but that several amendments and modifications thereof are possible without deviating from the scope of the present invention as defined by the attached claims. In particular, combinations of specific features of various aspects of the invention may be made. An aspect of the invention may be further advantageously enhanced by adding a feature that was described in relation to another aspect of the invention. While the present invention has been illustrated and described in detail in the figures and the description, such illustration and description are to be considered illustrative or exemplary only, and not restrictive.

The present invention is not limited to the disclosed embodiments. Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. The mere fact that certain measures are recited in mutually different dependent claims does not indicate that a combination of these measures cannot be used to advantage. Any reference numerals in the claims should not be construed as limiting the scope of the present invention.

### REFERENCE NUMERALS

- 10: System
- 11: Body Part
- 20: Housing
- 21: First Part of The Housing
- 22: Second Part of The Housing
- 23: Antenna
- 24: Sensors
- 25: Audio device
- 26: Processing Unit
- 27: Transducers
- 28: Electronic Module
- 30: Wired Connection
- 40: Energy Supply
- 50: Data exchanging unit

## Claims

1. A wearable monitoring and assistance system (10) for detecting at least one of physiological and biometrical data of a body of a user and/or for exciting the body locally with stimuli, the system (10) comprising:
- a housing (20) that is configured to be attached in use of said system to at least a part (11) of the body of the user, the housing (20) having an ergonomic shape that is configured to correspond to said part (11) of the body, the housing (20) comprising:
• a number of sensors (24) that are configured to detect said physiological and/or biometrical data of said part (11) of the body,
• a number of transducers (27) that are configured to excite said part (11) of the body locally with stimuli,
• a processing unit (26) that is operatively connected to the number of sensors (24) and the number of transducers (27), wherein the processing unit (26) is configured to generate a signal based on said physiological and/or biometrical data and is configured to generate stimuli for locally exciting said part (11) of the body,
• an antenna (23) that is adapted to receive the signal from the processing unit (26), and
• an electronic module (28) that is operatively connected with the number of sensors (24), the number of transducers (27), the processing unit (26) and the antenna (23),
- an energy supply (40) that is operatively connected with the electronic module (28), wherein the energy supply (40) is arranged outside of the housing (20),
wherein the housing (20) further comprises a first part (21) that is arranged outwardly protruding from said part (11) of the body and remaining within the perimeter of the said part (11) of the body, and wherein the antenna is integrally attached underneath of the first part (21) thereby maintaining the antenna (23) at a distance from said part (11) of the body and to avoid contact with said part (11) of the body..

2. The system (10) according to claim 1, further comprising a data exchanging unit (50) that is arranged outside of said part (11) of the body and is configured to wirelessly receive the signal from the processing unit (26) via the antenna (23), and wherein the data exchanging unit (50) is adapted to send an additional signal to the processing unit (26) via the antenna (23).

3. The system (10) according to claim 1 or 2, wherein the first part (21) of the housing (20) includes a grip structure that accommodates the antenna.

4. The system (10) according to anyone of the preceding claims, wherein the energy supply (40) is arranged outside of said part (11) of the body.

5. The system (10) according to anyone of the preceding claims, wherein the energy supply (40) comprises a battery carrier that is configured to be attached to said part (11) of the body.

6. The system according to claim 5, wherein the battery carrier is adapted to accommodate at least one battery that is adapted to be swappable.

7. The system (10) according to claim 5 or 6, wherein the energy supply (40) is adapted to wirelessly provide energy to the battery carrier.

8. The system (10) according to anyone of the claims 5-7, wherein the battery carrier is connected to the housing (20) via a wired connection system (30).

9. The system (10) according to claim 8, wherein the wired connection system (30) is adapted to transmit energy from the battery carrier to the housing (20) in the form of electrical current, magnetic induction or light.

10. The system (10) according to claim 1, wherein the energy supply (40) is adapted to wirelessly provide energy to the housing (20).

11. The system (10) according to anyone of the preceding claims, wherein the number of sensors (24) and the number of transducers (27) are detachable from the housing (20).

12. The system (10) according to anyone of the preceding claims, wherein the housing (20) has a second part (22) that in use of the system is arranged to be in contact with said part (11) of the body, wherein the second part (22) has an audio device (25).

13. The system (10) according to claim 12, wherein the second part (22) of the housing (20) has at least a part of the number of sensors (24) and of the number of transducers (27).

14. The system (10) according to claim 12 or 13, wherein the first part (21) of the housing (20) and the second part (22) of the housing (20) are arranged parallel to each other.

15. The system (10) according to anyone of the preceding claims, wherein said part (11) the body is configured to define concha of an ear.
